# EUROPEAN PATENT APPLICATION

(11) **EP 3 561 760 A1**
(43) Date of publication of application: **30.10.2019**
(21) Application number: 17883257.2
(22) Date of filing: 15.11.2017
(51) Int. Cl.: G06Q 50/22, G06Q 30/02

(54) **DISPLAY CONTROL DEVICE, DISPLAY CONTROL METHOD, AND COMPUTER PROGRAM**

(30) Priority: 22.12.2016 JP 2016248887
(71) Applicant: Sony Corporation, Tokyo 108-0075 (JP)
(72) Inventor: TAKATSUKA, Susumu, Tokyo 108-0075 (JP)
(74) Representative: MFG Patentanwälte Meyer-Wildhagen Meggle-Freund Gerhard PartG mbB
(86) International application number: PCT/JP2017/041077
(87) International publication number: WO 2018/116703

(57) **Abstract**

[Object] To provide a display control device capable of changing a presentation content of information obtained from a state of a user in accordance with an action of the user.

[Solution] Provided is a display control device including: an acquisition portion that acquires activity information of a user; and a display processing portion that performs processing for presenting first information obtained from a state of a user based on the activity information acquired by the acquisition portion and changing a content of the presented first information in accordance with an action of the user.

## Description

### Technical Field

The present disclosure relates to a display control device, a display control method, and a computer program.

### Background Art

It is important for a human to carry out a proper exercise and take a proper amount of rest for mental and physical health. For example, Patent literature 1 discloses a technique intended for a user to continuously execute an activity, such as an exercise, which is once started by allowing the user to maintain and improve the motivation for continuing the ongoing activity.

### Citation List

### Patent Literature

Patent Literature 1: JP 2006-131040A

### Disclosure of Invention

### Technical Problem

Patent literature 1 discloses the technique that allows the user to continuously execute the activity which is once started. However, above Patent literature 1 does not disclose a technique for urging the user in a certain state to change that state.

Thus, the present disclosure proposes a novel and improved display control device, display control method, and computer program capable of changing a presentation content of information obtained from a state of a user in accordance with an action of the user.

### Solution to Problem

According to the present disclosure, there is provided a display control device including: an acquisition portion that acquires activity information of a user; and a display processing portion that performs processing for presenting first information obtained from a state of a user based on the activity information acquired by the acquisition portion and changing a content of the presented first information in accordance with an action of the user.

In addition, according to the present disclosure, there is provided a display control method, including: acquiring activity information of a user; and performing, by a processor, processing for presenting first information obtained from a state of a user based on the acquired activity information and changing a content of the presented first information in accordance with an action of the user.

In addition, according to the present disclosure, there is provided a computer program for causing a computer to execute: acquiring activity information of a user; and performing processing for presenting first information obtained from a state of a user based on the acquired activity information and changing a content of the presented first information in accordance with an action of the user.

### Advantageous Effects of Invention

As described above, according to the present disclosure, it becomes possible to provide a novel and improved display control device, display control method, and computer program capable of changing the presentation content of the information obtained from the state of the user in accordance with the action of the user.

Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is an explanatory diagram illustrating an overall configuration example of a health management system 1 according to an embodiment of the present disclosure.
[FIG. 2] FIG. 2 is an explanatory diagram illustrating a functional configuration example of a collection device 100 according to the embodiment.
[FIG. 3] FIG. 3 is an explanatory diagram illustrating a functional configuration example of a portable terminal 200 according to the embodiment.
[FIG. 4] FIG. 4 is an explanatory diagram illustrating a functional configuration example of a server 300 according to the embodiment.
[FIG. 5] FIG. 5 is an explanatory diagram illustrating an example of a user interface.
[FIG. 6] FIG. 6 is an explanatory diagram illustrating an example of a user interface.
[FIG. 7] FIG. 7 is an explanatory diagram illustrating an example of a user interface.
[FIG. 8] FIG. 8 is an explanatory diagram illustrating an operation example of the portable terminal 200 according to the embodiment.
[FIG. 9A] FIG. 9A is an explanatory diagram illustrating an example of a user interface.
[FIG. 9B] FIG. 9B is an explanatory diagram illustrating an example of a user interface.
[FIG. 9C] FIG. 9C is an explanatory diagram illustrating an example of a user interface.
[FIG. 9D] FIG. 9D is an explanatory diagram illustrating an example of a user interface.
[FIG. 10A] FIG. 10A is an explanatory diagram illustrating an example of a user interface.
[FIG. 10B] FIG. 10B is an explanatory diagram illustrating an example of a user interface.
[FIG. 10C] FIG. 10C is an explanatory diagram illustrating an example of a user interface.
[FIG. 10D] FIG. 10D is an explanatory diagram illustrating an example of a user interface.
[FIG. 11] FIG. 11 is an explanatory diagram illustrating an operation example of the portable terminal 200 according to the embodiment.
[FIG. 12A] FIG. 12A is an explanatory diagram illustrating an example of a user interface.
[FIG. 12B] FIG. 12B is an explanatory diagram illustrating an example of a user interface.
[FIG. 12C] FIG. 12C is an explanatory diagram illustrating an example of a user interface.
[FIG. 12D] FIG. 12D is an explanatory diagram illustrating an example of a user interface.
[FIG. 13A] FIG. 13A is an explanatory diagram illustrating an example of a user interface.
[FIG. 13B] FIG. 13B is an explanatory diagram illustrating an example of a user interface.
[FIG. 14A] FIG. 14A is an explanatory diagram illustrating an example of a user interface.
[FIG. 14B] FIG. 14B is an explanatory diagram illustrating an example of a user interface.
[FIG. 15] FIG. 15 is an explanatory diagram illustrating an example in which an item of a user is represented by a point system.
[FIG. 16] FIG. 16 is an explanatory diagram illustrating a correspondence relation.
[FIG. 17] FIG. 17 is an explanatory diagram illustrating a correspondence relation.
[FIG. 18] FIG. 18 is an explanatory diagram illustrating an example of a change in points of the user and a content presented by the portable terminal 200 in accordance with the change in the points of the user.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that description will be provided in the following order.
1. Embodiment of present disclosure
   1.1. Outline
   1.2. System configuration example
   1.3. Functional configuration example
   1.4. Operation example and display example
2. Summary

### <1. Embodiment of present disclosure>

### [1.1. Outline]

An outline of an embodiment of the present disclosure will be described before the embodiment of the present disclosure is described in detail.

It is important for a human to carry out a proper exercise and take a proper amount of rest for mental and physical health. A human repeats a sitting state, a standing state, an exercise state, and the like throughout the day. Such being the case, the person's health condition is progressively deteriorated as the time of the sitting state becomes longer. For example, if the person who takes sufficient exercise continues to sit on the chair in a non-exercise time, the person's health condition is deteriorated due to the prolonged time of the sitting state.

Further, there are studies that have reported that taking a proper amount of rest during work leads to the enhancement of productivity.

Thus, when a person continues to be in a certain activity state for a long period of time, urging the person to switch (change) the ongoing activity state to another state on a regular basis can not only enhance the productivity of the person, but also potentially improve the mental and physical health conditions of the person.

Thus, in view of the foregoing points, the present disclosing party has intensively studied a technique that can detect a state of a user and, upon determining that a certain state is continued for a predetermined time, urge the user to switch (change) the current state. As a result, the present disclosing party has invented a technique that can detect a state of a user and, upon determining that a certain state is continued for a predetermined time, urge the user to switch (change) the current state. Such a technique will be described below.

The outline of the embodiment of the present disclosure has been described above.

### [1.2. System configuration example]

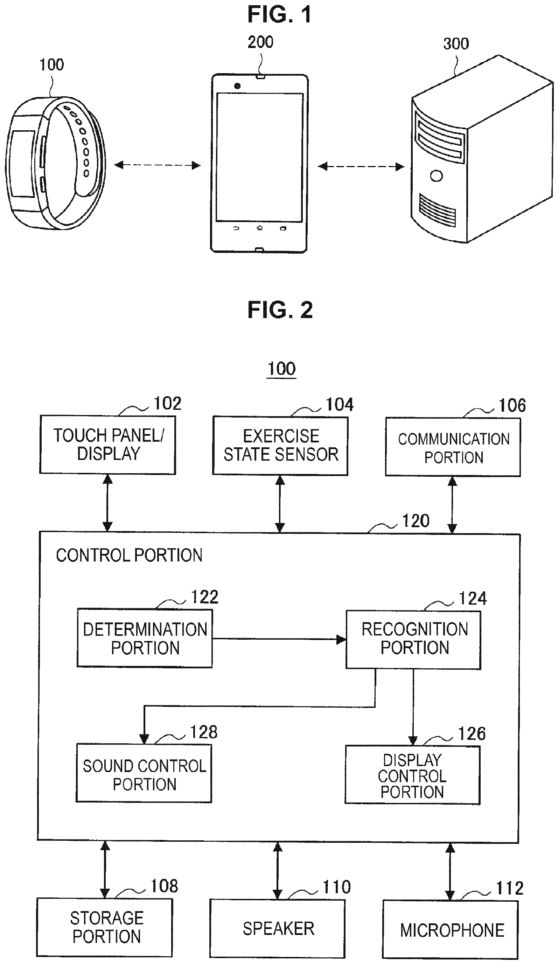

Next, a configuration example of a system according to the embodiment of the present disclosure will be described. FIG. 1 is an explanatory diagram illustrating an overall configuration example of a health management system 1 according to the embodiment of the present disclosure. The overall configuration example of the health management system 1 according to the embodiment of the present disclosure will be described below using FIG. 1.

As shown in FIG. 1, the health management system 1 according to the embodiment of the present disclosure is configured by including a collection device 100, a portable terminal 200, and a server 300.

The collection device 100 is a device that collects information regarding the body of a user and can be put, for example, on the body of the user, particularly, on the wrist. The collection device 100 can be formed, for example, in a shape of a wearable device. In FIG. 1, the collection device 100 is shown as a wristwatch-type wearable device intended to be put on the wrist of the user. However, the shape of the collection device 100 is not limited to that of the wristwatch-type wearable device.

The collection device 100 collects information regarding the body of the user such as, for example, the heart rate and heartbeat variability of the user. For example, the collection device 100 captures a change in a blood flow by radiating LED (Light Emitting Diode) light to the skin of the user and measures a pulse wave of the user from the change in the blood flow. The collection device 100 can collect the information regarding the body of the user such as the heart rate and heartbeat variability of the user by measuring the pulse wave of the user.

Further, the collection device 100 provided with an acceleration sensor collects sensing data outputted from the acceleration sensor.

The portable terminal 200 is a terminal with a communication function carried by the user. The portable terminal 200 can be formed, for example, in a shape of a smartphone, a tablet terminal, or the like. The portable terminal 200 acquires the information regarding the body of the user and the sensing data from the collection device 100 via wireless communication with the collection device 100. Further, if the state of the user is continued for a predetermined time on the basis of the information and data thus acquired, the portable terminal 200 has a function of presenting information for changing the ongoing state.

Note that various kinds of information from the collection device 100 may be received by a laptop or desktop personal computer instead of the portable terminal 200.

The server 300 is a device that acquires information from the portable terminal 200 and provides information to the portable terminal 200 via communication with the portable terminal 200. The server 300 may be regarded as a single device by the portable terminal 200 by having a configuration in which a plurality of devices is connected to each other via a network. The server 300 acquires the information regarding the body of the user and the sensing data acquired by the portable terminal 200 and, if the state of the user is continued for a predetermined time, provides information for presenting information for changing the ongoing state to the portable terminal 200.

The portable terminal 200 (or the server 300) can precisely estimate the activity state of the user by acquiring the information regarding the body of the user and the sensing data from the collection device 100. The portable terminal 200 can estimate the state of the user, whether the user is in a state of resting, sitting, standing, carrying out a moderate exercise, carrying out a vigorous exercise, or the like, and also measure a duration time of each state using the information regarding the body of the user and the sensing data.

Note that FIG. 1 shows an example in which the health management system 1 according to the embodiment of the present disclosure is configured by including the collection device 100, the portable terminal 200, and the server 300. However, the present disclosure is not limited to such an example. Processing described below may be performed by the collection device 100 alone, the portable terminal 200 alone, a combination of the collection device 100 and the portable terminal 200, a combination of the collection device 100 and the server 300, or a combination of the portable terminal 200 and the server 300.

The above describes the overall configuration example of the health management system 1 according to the embodiment of the present disclosure. Next, a functional configuration example of each device according to the embodiment of the present disclosure will be described.

### [1.3. Functional configuration example]

FIG. 2 is an explanatory diagram illustrating a functional configuration example of the collection device 100 according to the embodiment of the present disclosure. The functional configuration example of the collection device 100 according to the embodiment of the present disclosure will be described below using FIG. 2.

As shown in FIG. 2, the collection device 100 according to the present embodiment is configured by including a touch panel display 102, an exercise state sensor 104, a communication portion 106, a storage portion 108, a speaker 110, a microphone 112, and a control portion 120. Further, the control portion 120 is configured by including a determination portion 122, a recognition portion 124, a display control portion 126, and a sound control portion 128. Note that each constituting element of the collection device 100 shown in FIG. 2 can be constitute by a hardware, or a central processing unit such as CPU and a program for causing the central processing unit to perform the function. Note that the collection device 100 may be free from the touch panel display 102, the communication portion 106, the speaker 110, and the microphone 112.

The touch panel display 102 is a display device including a touch panel and configured to be able to detect a contact of a finger of the user or the like.

The exercise state sensor 104 includes, for example, a heart rate sensor and an acceleration sensor. The sensing data outputted by the exercise state sensor 104 are transmitted to the control portion 120.

The communication portion 106 executes transmission/reception of information by performing communication with other devices under the control of the control portion 120. The communication portion 106 is configured by including a hardware having a communication function, for example, a communication circuit. The storage portion 108 is constituted by, for example, ROM, a flash memory, or the like and stores a computer program operated in the collection device 100, various kinds of information, and the like.

The determination portion 122 of the control portion 120 determines whether the user is in a sitting state, a standing state, a state of carrying out a moderate exercise, a state of carrying out a vigorous exercise, or the like from a detection value of the exercise state sensor 104. The determination portion 122 can also determine the time when the user state changes from the sitting state to the standing state and the number of times the user state changes from the sitting state to the standing state within a predetermined time (e.g., per day). The recognition portion 124 recognizes the state of the user and also a continuous time in which the user has been in that state on the basis of the determination result of the determination portion 122.

The display control portion 126 performs processing for displaying processing results of the determination portion 122 and the recognition portion 124 on the touch panel display 102. The sound control portion 128 performs processing for outputting via sound the processing results of the determination portion 122 and the recognition portion 124 from the speaker 110. Note that exercise state sensor 104 may include a sensor that detects other biological information such as a body temperature sensor and the determination portion 122 may determine the exercise state using multiple pieces of biological information such as the heartbeat and the body temperature.

The above describes the functional configuration example of the collection device 100 according to the embodiment of the present disclosure. Next, a functional configuration example of the portable terminal 200 according to the embodiment of the present disclosure will be described.

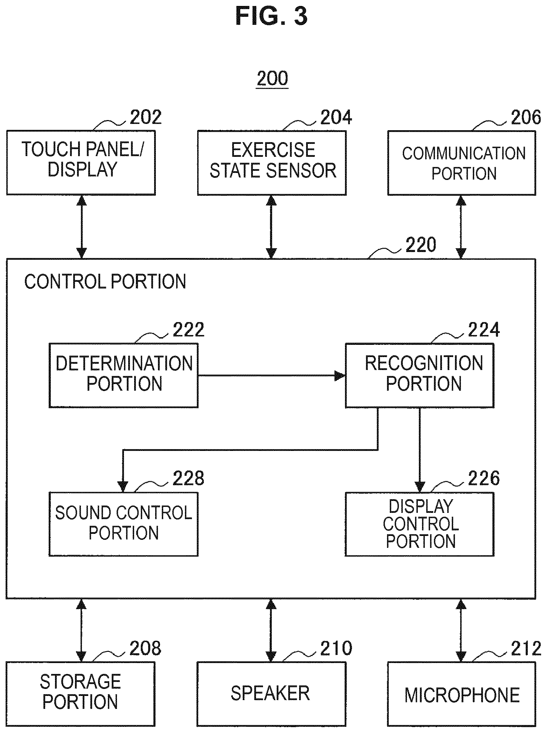

FIG. 3 is an explanatory diagram illustrating a functional configuration example of the portable terminal 200 according to the embodiment of the present disclosure. The functional configuration example of the portable terminal 200 according to the embodiment of the present disclosure will be described below using FIG. 3.

As shown in FIG. 3, the portable terminal 200 according to the present embodiment is configured by including a touch panel display 202, an exercise state sensor 204, a communication portion 206, a storage portion 208, a speaker 210, a microphone 212, and a control portion 220. Further, the control portion 220 is configured by including a determination portion 222, a recognition portion 224, a display control portion 226, and a sound control portion 228. Note that each constituting element of the portable terminal 200 shown in FIG. 3 can be constitute by a hardware, or a central processing unit such as CPU and a program for causing the central processing unit to perform the function.

The touch panel display 202 is a display device including a touch panel and configured to be able to detect a contact of a finger of the user or the like.

The exercise state sensor 204 includes, for example, a heart rate sensor and an acceleration sensor. The sensing data outputted by the exercise state sensor 204 are transmitted to the control portion 220.

The communication portion 206, which is an example of an acquisition portion of the present disclosure, executes transmission/reception of information by performing communication with other devices under the control of the control portion 220. The communication portion 206 is configured by including a hardware having a communication function, for example, a communication circuit. The storage portion 208 is constituted by, for example, ROM, a flash memory, or the like and stores a computer program operated in the collection device 200, various kinds of information, and the like.

The determination portion 222 of the control portion 220 determines whether the user is in a sitting state, a standing state, a state of carrying out a moderate exercise, a state of carrying out a vigorous exercise, or the like from a detection value of the exercise state sensor 204. The determination portion 222 can also determine the time when the user state changes from the sitting state to the standing state and the number of times the user state changes from the sitting state to the standing state within a predetermined time (e.g., per day). Further, the determination portion 222 can determine how long the user has been in the ongoing state from the detection value of the exercise state sensor 204 and the information acquired from the collection device 100, for example, the biological information of the user wearing the collection device 100. The recognition portion 224 recognizes the state of the user and also the continuous time in which the user has been in that state on the basis of the determination result of the determination portion 222.

The display control portion 226, which is an example of the display processing portion of the present disclosure, performs processing for displaying processing results of the determination portion 222 and the recognition portion 224 on the touch panel display 202. In the present embodiment, the display control portion 226 outputs information for changing the state of the user determined by the determination portion 222. A specific output example of the information will be described below. The sound control portion 228 performs processing for outputting via sound the processing results of the determination portion 222 and the recognition portion 224 from the speaker 210. Note that exercise state sensor 204 may include a sensor that detects other biological information such as a body temperature sensor and the recognition portion 224 may determine the exercise state using multiple pieces of biological information such as the heartbeat and the body temperature.

The above describes the functional configuration example of the portable terminal 200 according to the embodiment of the present disclosure. Next, a functional configuration example of the server 300 according to the embodiment of the present disclosure will be described.

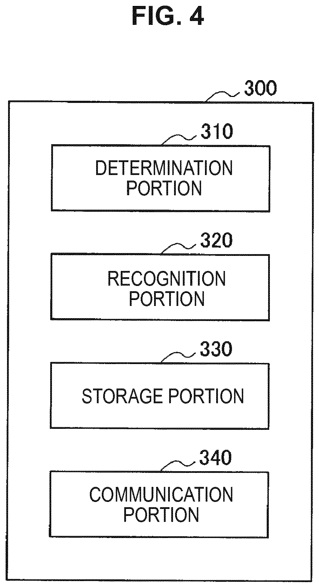

FIG. 4 is an explanatory diagram illustrating aa functional configuration example of the server 300 according to the embodiment of the present disclosure. The functional configuration example of the server 300 according to the embodiment of the present disclosure will be described below using FIG. 4.

As shown in FIG. 4, the server 300 according to the embodiment of the present disclosure is configured by including a determination portion 310, a recognition portion 320, a storage portion 330, and a communication portion 340.

The determination portion 310 determines whether the user is in a sitting state, a standing state, a state of carrying out a moderate exercise, a state of carrying out a vigorous exercise, or the like from the detection value of the exercise state sensor 104 (or the exercise state sensor 204) which is transmitted from the collection device 100 or the portable terminal 200 and received by the communication portion 340. The determination portion 222 can also determine the time when the user state changes from the sitting state to the standing state and the number of times the user state changes from the sitting state to the standing state within a predetermined time (e.g., per day). The recognition portion 320 recognizes the state of the user and also the continuous time in which the user has been in that state on the basis of the determination result of the determination portion 310.

The storage portion 330 is constituted by, for example, ROM, a flash memory, HDD, or the like and stores a computer program operated in the server 300, various kinds of information, and the like. The communication portion 340 executes transmission/reception of information by performing communication with other devices. The communication portion 340 is configured by including a hardware having a communication function, for example, a communication circuit.

The above describes the functional configuration example of the server 300 according to the embodiment of the present disclosure. Next, an operation example of the health management system 1 according to the embodiment of the present disclosure will be described.

### [1.4. Operation example and display example]

A description below is given of a case where the portable terminal 200 displays the state of the user wearing the collection device 100 on the body (hereinafter also simply referred to as "user"). An example of a user interface in an application to be executed by the portable terminal 200 will be described before an operation example of the health management system 1 according to the present embodiment is described in detail.

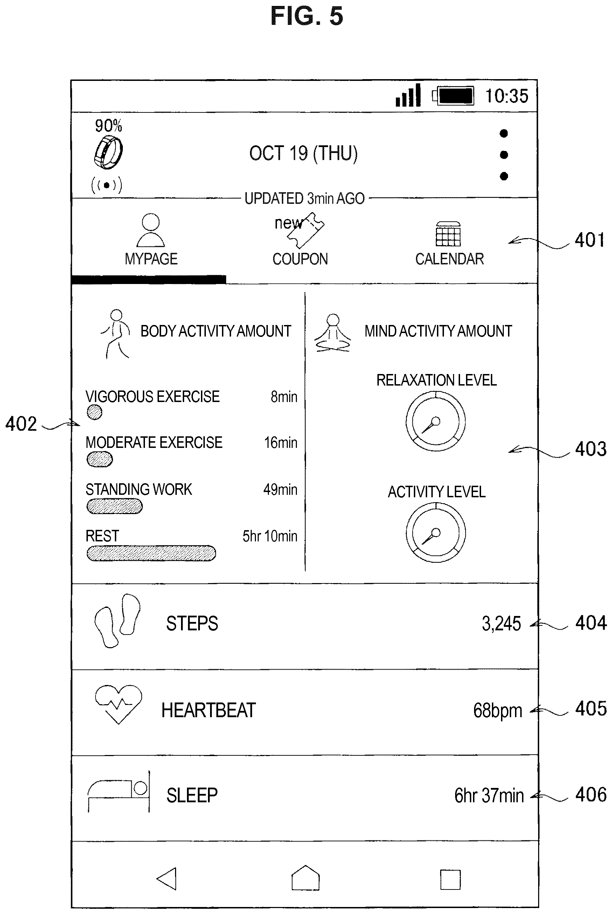

FIGS. 5 to 7 are explanatory diagrams each illustrating an example of the user interface in the application to be executed by the portable terminal 200 according to the present embodiment. The application to be executed by the portable terminal 200 according to the present embodiment includes a screen displaying the state of the user wearing the collection device 100 on the body, a screen displaying a coupon, an earned point, or the like provided on the basis of the action of the user wearing the collection device 100 on the body, a screen displaying an event related to the user wearing the collection device 100 on the body, and the like.

FIG. 5 is an explanatory diagram illustrating an example of a screen displaying the state of the user wearing the collection device 100 on the body. The screen shown in FIG. 5 includes regions 401 to 406. The region 401 is a region for switching screens. When the user touches "MyPage" in the region 401, the screen of the portable terminal 200 is switched to the one shown in FIG. 5. The region 402 is a region for displaying information on a physical activity amount (a body activity amount) of the user wearing the collection device 100 on the body. The region 403 is a region for displaying information on a mental activity amount (a mind activity amount) of the user. The region 404 is a region for displaying the number of steps taken by the user. The region 405 is a region for displaying the heart rate of the user. The region 406 is a region for displaying a sleeping time of the user.

Any information displayed in the regions 402 to 406 is consequently presented after the determination portion 222 determines the state on the basis of the information collected by the collection device 100 and the recognition portion 224 recognizes the duration time, the number of times, and the like.

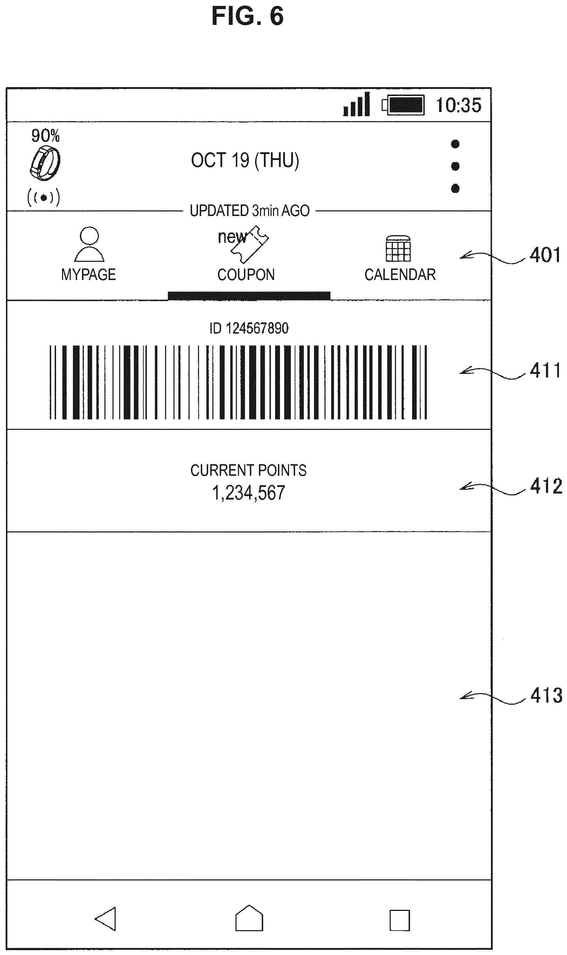

FIG. 6 is an explanatory diagram illustrating an example of a screen displaying a coupon, an earned point, or the like provided on the basis of the action of the user. The screen shown in FIG. 6 includes the regions 401 and 411 to 413. When the user touches "Coupon" in the region 401, the screen of the portable terminal 200 is switched to the one shown in FIG. 6. The region 411 is a region for displaying information for identifying the user (ID, a barcode, etc.). The region 412 is a region for displaying the current earned point of the user. The region 413 is a region for displaying a reward such as the coupon provided on the basis of the action of the user.

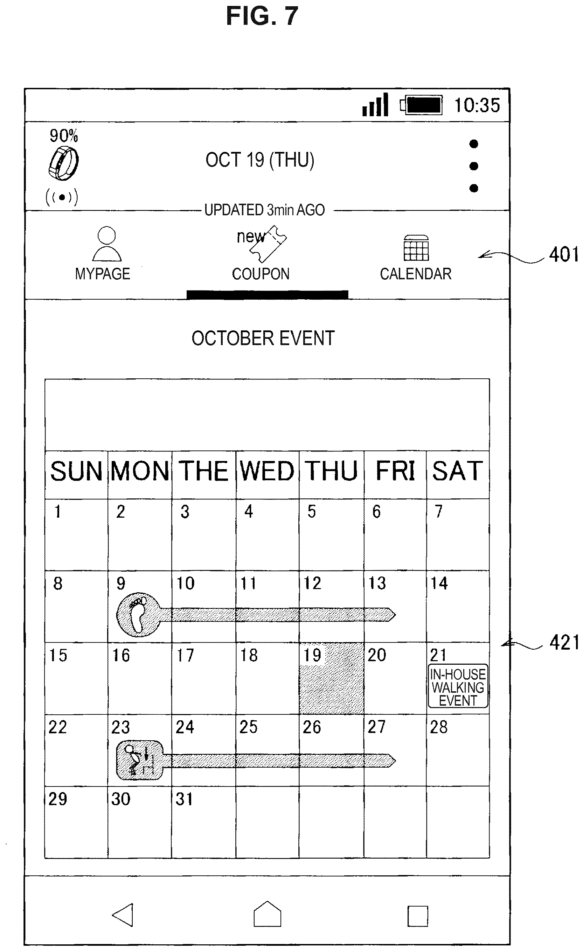

FIG. 7 is an explanatory diagram illustrating an example of a screen displaying an event related to the user. The screen shown in FIG. 7 includes regions 401 and 421. When the user touches "Calendar" in the region 401, the screen of the portable terminal 200 is switched to the one shown in FIG. 7. The region 421 is a region for displaying the event related to the user in a calendar format.

A description provided below mainly focuses on a display control for displaying the screen shown in FIG. 6 for urging the user to change the current action (a health promoting action proposal screen) in order to provide the reward such as the coupon and the earned point on the basis of the action of the user.

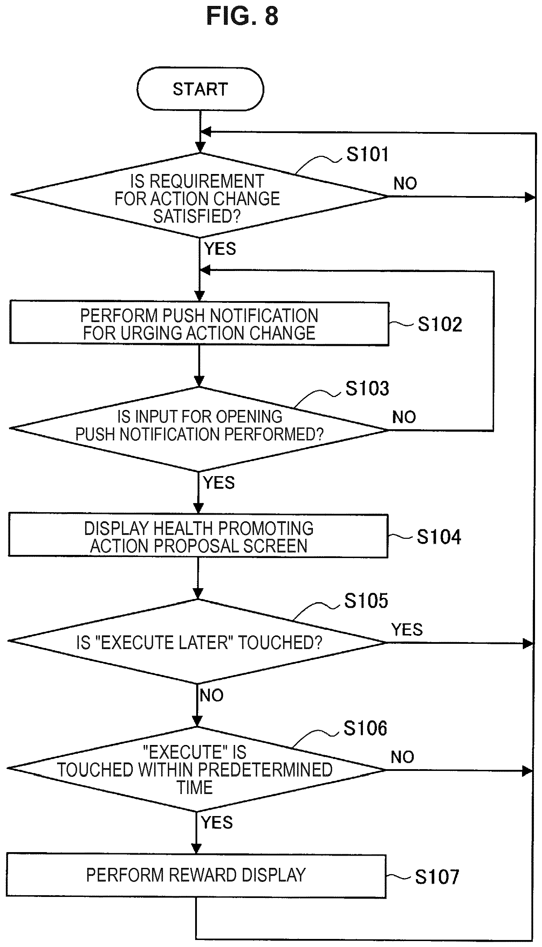

FIG. 8 is an explanatory diagram illustrating an operation example of the portable terminal 200 according to the embodiment of the present disclosure. The operation example of the portable terminal 200 according to the present embodiment will be described below using FIG. 8.

The portable terminal 200 periodically acquires the information from the collection device 100 and determines whether the acquired information satisfies a requirement for action change (a step SI01). This determination in the step S101 is executed by, for example, the determination portion 222.

Examples of the requirement for action change include the sitting action being continued for a predetermined time, the tensed state being continued for a predetermined time, the vigorous exercise being continued for a predetermined time, the increased number of steps per predetermined time being a predetermined step number or less, the sleeping time being less than a predetermined time, a predetermined time being reached after waking up, and the like.

The portable terminal 200 waits until the information acquired from the collection device 100 satisfies the requirement for action change (No in the step S101) and, if the information acquired from the collection device 100 satisfies the requirement for action change (Yes in the step SI01), the portable terminal 200 performs a push notification for urging the action change (a step S102). An example of the push notification will be described below. Then, the portable terminal 200 determines whether an input for opening the push notification is performed (a step S103).

The portable terminal 200 waits until the input for opening the push notification is performed (No in the step S103) and, if the input for opening the push notification is performed (Yes in the step S103), the portable terminal 200 displays the health promoting action proposal screen (a step S104). A display example of the health promoting action proposal screen will be described in detail below.

The portable terminal 200 determines whether an "Execute Later" button displayed on the health promoting action proposal screen is touched (a step S105). If the "Execute Later" button is touched (Yes in the step S105), the portable terminal 200 returns to the determination processing of whether the requirement for action change is satisfied in the step S101. If the "Execute Later" button is not touched (No in the step S105), the portable terminal 200 subsequently determines whether an "Execute" button is touched within a predetermined time (a step SI06).

If the "Execute" button is not touched within a predetermined time (No in the step S106), the portable terminal 200 returns to the determination processing of whether the requirement for action change is satisfied in the step S101. If the "Execute" button is touched within a predetermined time (Yes in the step S106), the portable terminal 200 performs the reward display for providing the reward to the user (a step SI07).

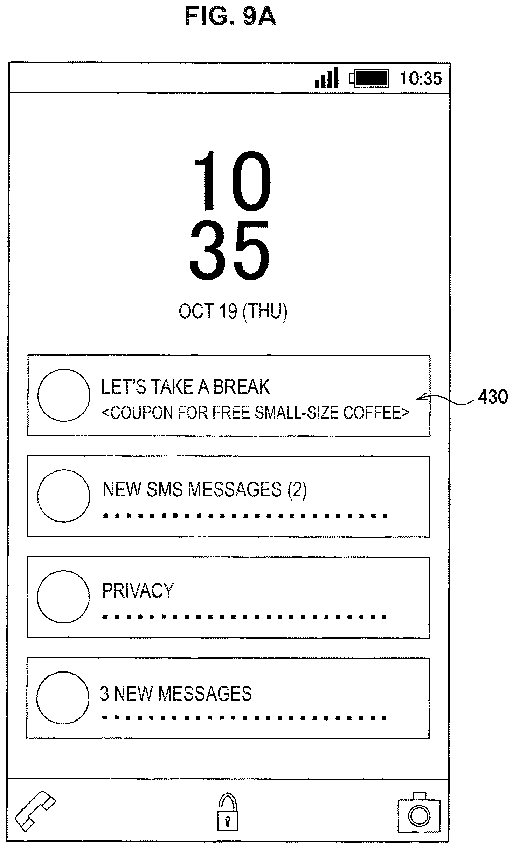

FIGS. 9A to 9D are explanatory diagrams each illustrating an example of the user interface displayed on the portable terminal 200 by a series of operations in FIG. 8. Here, a description is given of an example where a fifteen-minute walk is proposed to the user when it is detected that the sitting state of the user is continued for a predetermined time.

FIG. 9A shows an example of a screen that is displayed on the portable terminal 200 when the information acquired from the collection device 100 satisfies the requirement for action change and the portable terminal 200 performs the push notification for urging the action change. The push notification can be displayed on a lock screen when the user is not operating the portable terminal 200 or on a screen being operated when the user is operating the portable terminal 200. FIG. 9A shows how the push notification represented by reference numeral 430 is displayed on the portable terminal 200. Note that the push notification is not necessarily performed by the screen display. The portable terminal 200 may perform the push notification by a sound or vibration.

A method of the push notification may be selected by the user through an application setting. For example, whether the notification is performed through the screen display, a sound output from a speaker, or vibration of a vibrator may be selected both in the collection device 100 and the portable terminal 200. Further, the method of the push notification may change depending on a distance relationship between the collection device 100 and the portable terminal 200. For example, in a case where the user wears the collection device 100 and the collection device 100 is distanced from the portable terminal 200 by a predetermined value or greater, the notification may be performed only in the collection device 100.

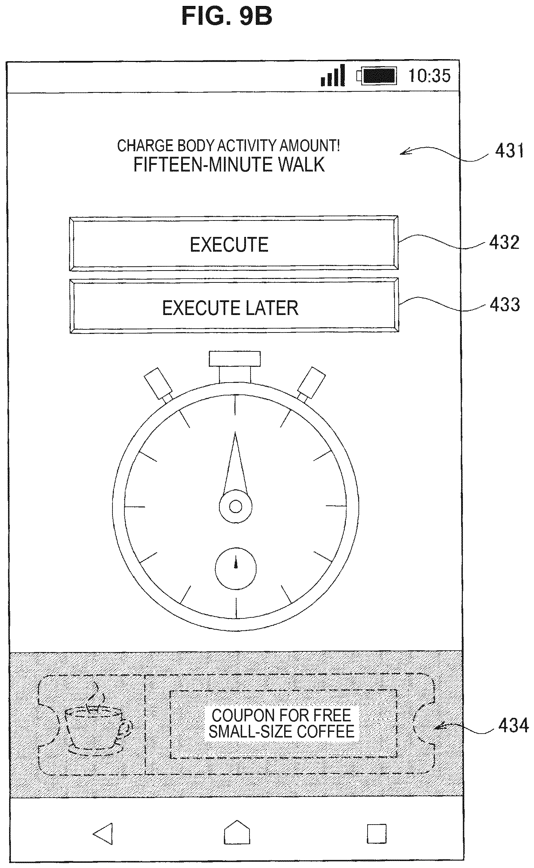

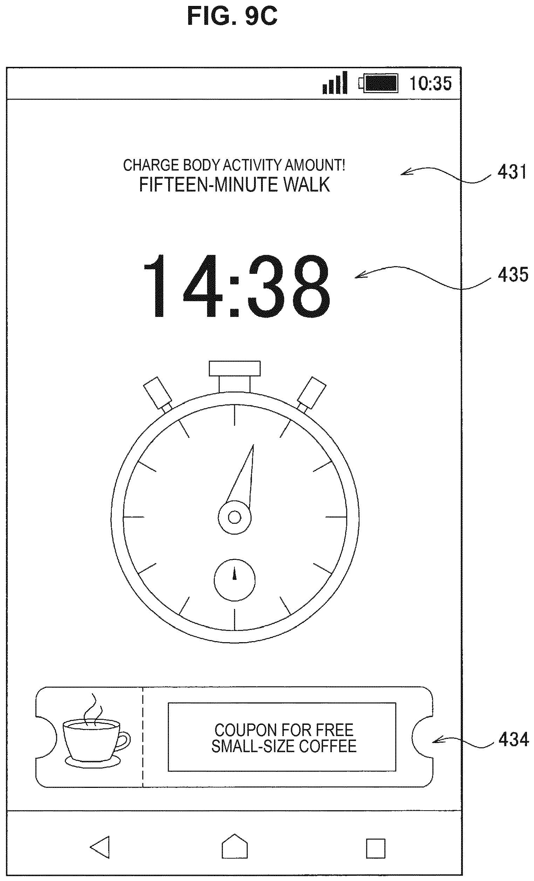

FIG. 9B is a display example of the health promoting action proposal screen performed in the step S104 in FIG. 8. The health promoting action proposal screen shown in FIG. 9B displays a region 431 displaying an action to be desirably performed by the user, a button 432 displayed as "Execute", a button 433 displayed as "Execute Later", and a coupon image 434. In this example, a fifteen-minute walk is presented in the region 431 as the action to be desirably performed by the user. The information presented in the region 431 is intended to change the state of the user determined by the determination portion 222. For example, if the determination portion 222 determines that the user continues the sitting state for two hours, a fifteen-minute walk is presented in the region 431 as the action to be desirably performed. If the user touches the button 432, the portable terminal 200 displays a screen shown in FIG. 9C described below. If the user touches the button 433, the health promoting action proposal screen on the portable terminal 200 is switched to another screen.

The coupon image 434 is translucently displayed on the portable terminal 200 at this point. The coupon image 434 may be made completely invisible on the portable terminal 200 at this point. Upon detecting that the user has executed the action presented in the region 431, the portable terminal 200 clearly displays the coupon image 434 as in the screen shown in FIG. 9C described below.

FIG. 9C is an example of the reward display performed in the step S107 in FIG. 8 and an example of the screen that is displayed on the portable terminal 200 when the user touches the button 433 in FIG. 8. The screen shown in FIG. 9C displays the region 431 displaying the action to be desirably performed by the user, a region 435 displaying a remaining time, and the coupon image 434. As the time displayed in the region 435 passes (as the remaining time decreases), a position of the hand on the stop watch displayed on the screen changes. Further, the portable terminal 200 clearly displays the coupon image 434.

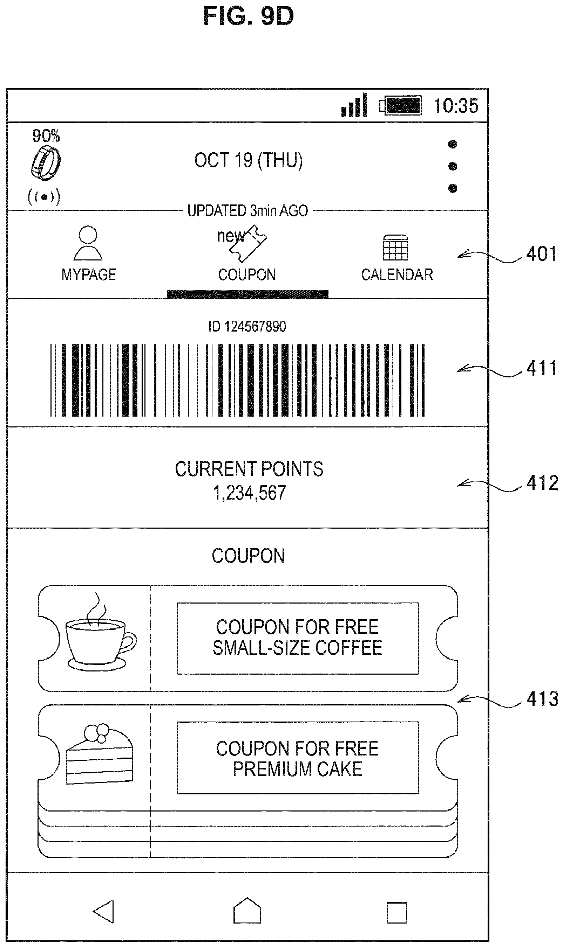

After the elapse of 15 minutes, as shown in FIG. 9D, the portable terminal 200 displays the coupon to be provided to the user for the fifteen-minute walk in the region 413.

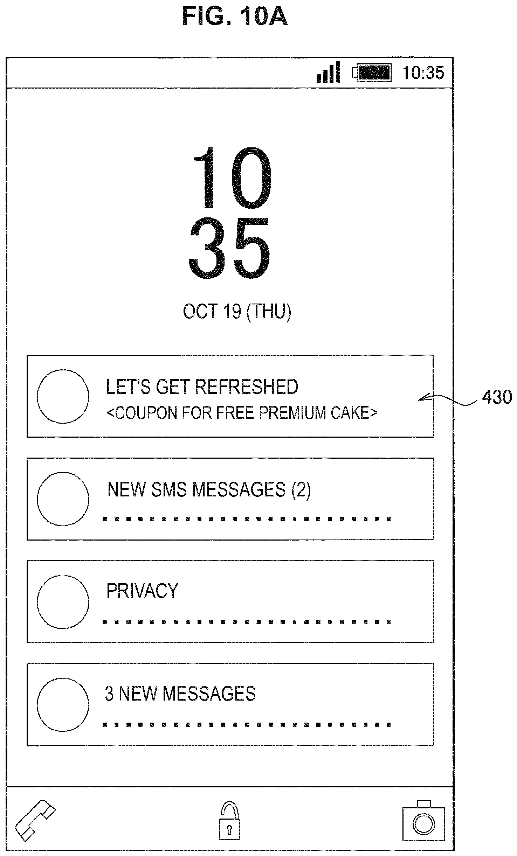

Another example of the screen displayed on the portable terminal 200 by the series of operations in FIG. 8 will be described. FIGS. 10A to 10D are explanatory diagrams each illustrating an example of the user interface displayed on the portable terminal 200 by the series of operations in FIG. 8. Here, a description is given of an example where a three-minute deep breath is proposed to the user when it is detected that the tensed state of the user is continued for a predetermined time.

FIG. 10A shows an example of a screen that is displayed on the portable terminal 200 when the information acquired from the collection device 100 satisfies the requirement for action change and the portable terminal 200 performs the push notification for urging the action change. FIG. 10A shows how the push notification represented by reference numeral 430 is displayed on the portable terminal 200.

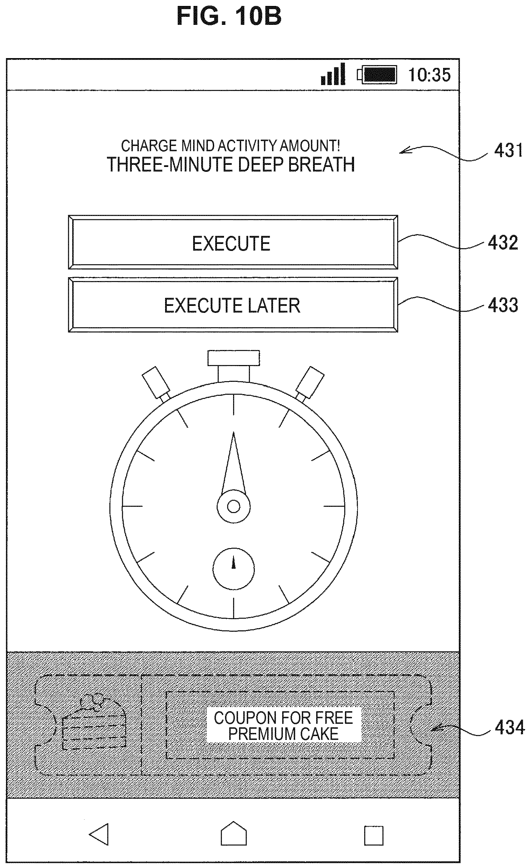

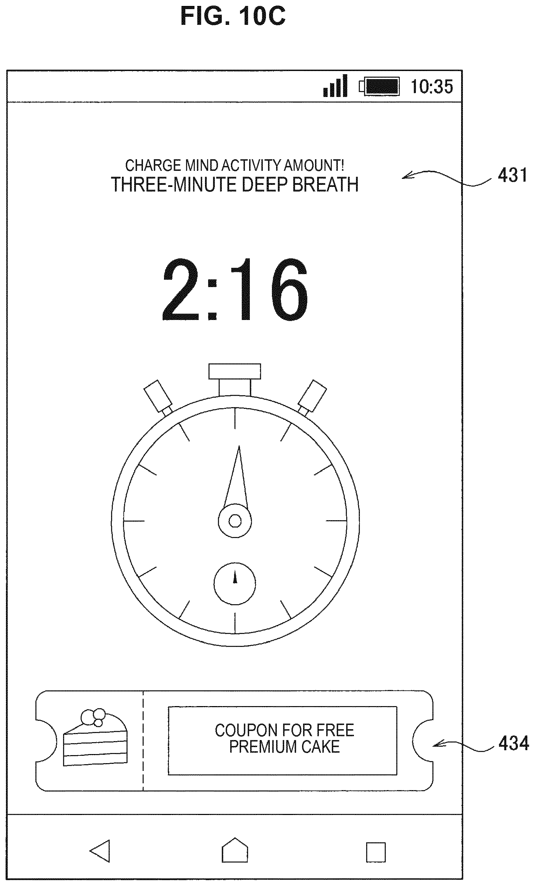

FIG. 10B is a display example of the health promoting action proposal screen performed in the step S104 in FIG. 8. The health promoting action proposal screen shown in FIG. 10B displays the region 431 displaying the action to be desirably performed by the user, the button 432 displayed as "Execute", the button 433 displayed as "Execute Later", and the coupon image 434. In this example, a three-minute deep breath is presented in the region 431 as the action to be desirably performed by the user. If the user touches the button 432, the portable terminal 200 displays a screen shown in FIG. 10C described below. If the user touches the button 433, the health promoting action proposal screen on the portable terminal 200 is switched to another screen.

FIG. 10C is an example of the reward display performed in the step S107 in FIG. 8 and an example of the screen that is displayed on the portable terminal 200 when the user touches the button 433 in FIG. 8. The screen shown in FIG. 9C displays the region 431 displaying the action to be preferably performed by the user, the region 435 displaying a remaining time, and the coupon image 434. As the time displayed in the region 435 passes (as the remaining time decreases), a position of the hand on the stop watch displayed on the screen changes. Further, the portable terminal 200 clearly displays the coupon image 434.

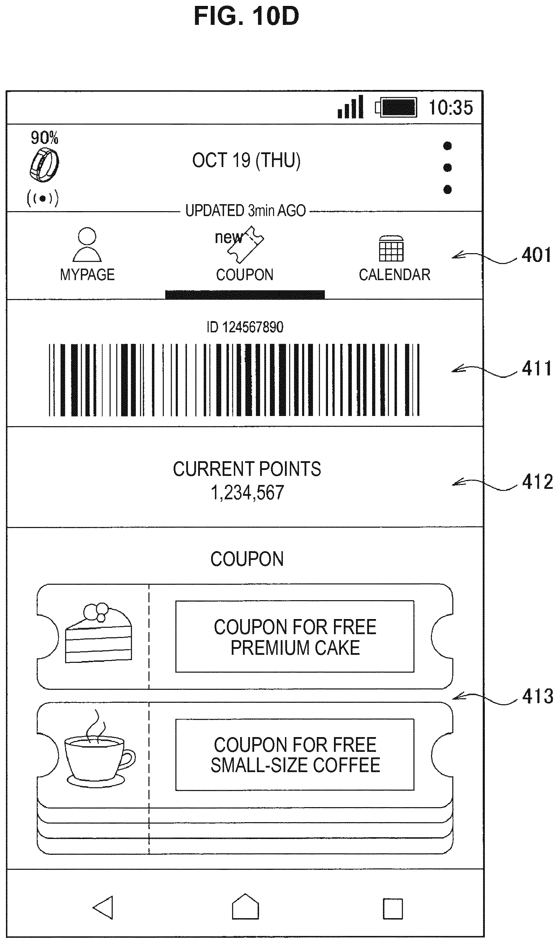

Then, when three minutes have passed since the user touched the button 433, as shown in FIG. 10D, the portable terminal 200 displays the coupon to be provided to the user for the three-minute deep breath in the region 413.

In this manner, the portable terminal 200 can present the action for changing the state of the user on the basis of the information transmitted from the collection device 100.

The portable terminal 200 may determine whether the presented action is performed by the user and provide the coupon or the earned point to the user only if the presented action is actually performed by the user.

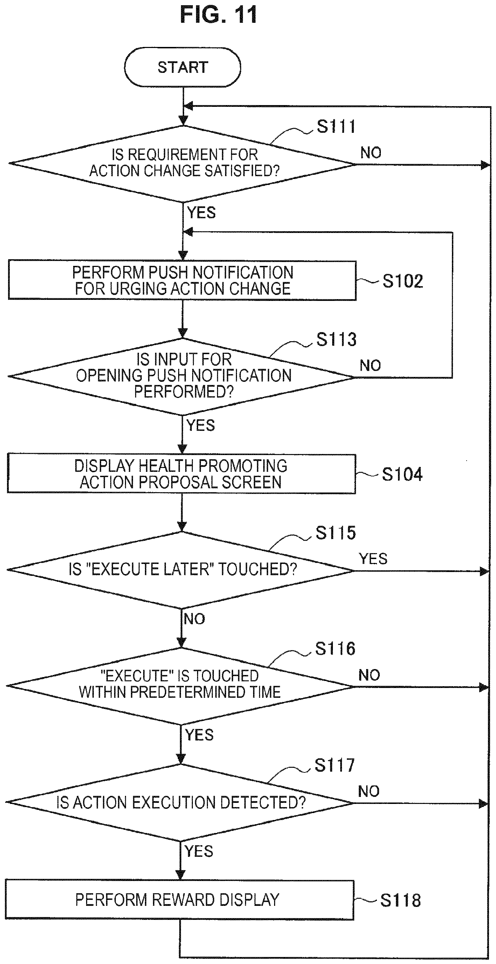

FIG. 11 is an explanatory diagram illustrating an operation example of the portable terminal 200 according to the embodiment of the present disclosure. The operation example of the portable terminal 200 according to the present embodiment will be described below using FIG. 11.

The portable terminal 200 periodically acquires the information from the collection device 100 and determines whether the acquired information satisfies the requirement for action change (a step S111). This determination in the step S101 is executed by, for example, the determination portion 222.

The portable terminal 200 waits until the information acquired from collection device 100 satisfies the requirement for action change (No in the step S111) and, if the information acquired from collection device 100 satisfies the requirement for action change (Yes in the step S111), the portable terminal 200 performs the push notification for urging the action change (a step S112). Then, the portable terminal 200 determines whether an input for opening the push notification is performed (a step S113).

The portable terminal 200 waits until the input for opening the push notification is performed (No in the step S123) and, if the input for opening the push notification is performed (Yes in the step S113), the portable terminal 200 displays the health promoting action proposal screen (a step S114).

The portable terminal 200 determines whether the "Execute Later" button displayed on the health promoting action proposal screen is touched (a step S115). If the "Execute Later" button is touched (Yes in the step S115), the portable terminal 200 returns to the determination processing of whether the requirement for action change is satisfied in the step Sill. If the "Execute Later" button is not touched (No in the step S115), the portable terminal 200 subsequently determines whether the "Execute" button is touched within a predetermined time (a step S116).

If the "Execute" button is not touched within the predetermined time (No in the step S116), the portable terminal 200 returns to the determination processing of whether the requirement for action change is satisfied in the step S111. If the "Execute" button is touched within the predetermined time (Yes in the step S116), the portable terminal 200 determines whether the presented action is actually executed (a step 117). Processing in the step S117 is executed by, for example, the determination portion 222. Further, the portable terminal 200 determines whether the presented action is actually executed on the basis of the information transmitted from the collection device 100. For example, the portable terminal 200 may determine that the user has not performed the deep breath yet if the heart rate per minute is a predetermined value or more even after the deep breath is presented.

If the presented action is not actually executed (No in the step S117), the portable terminal 200 returns to the determination processing of whether the requirement for action change is satisfied in the step S111. If the presented action is actually executed (Yes in the step S117), the portable terminal 200 performs the reward display for providing the reward to the user (a step S118).

When the presented activity is actually executed and the coupon or the earned point is provided to the user in this manner, the portable terminal 200 may change the display of the coupon in accordance with an execution degree of the action.

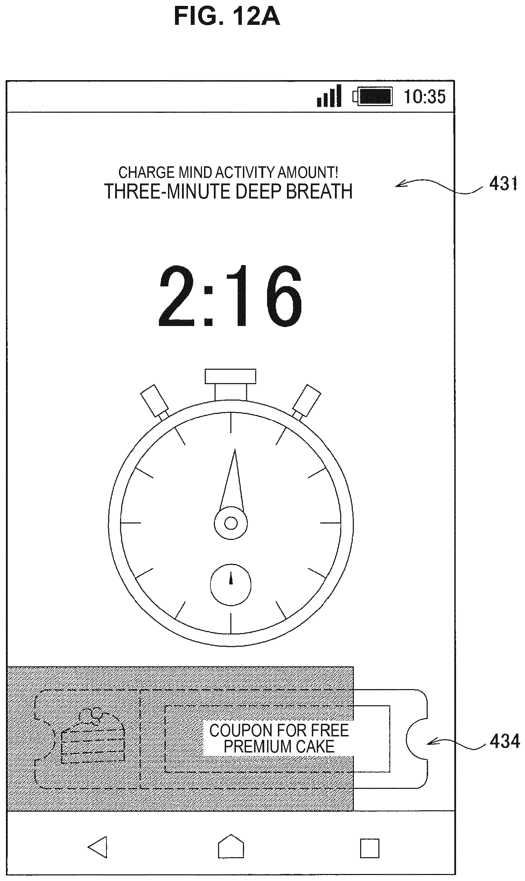

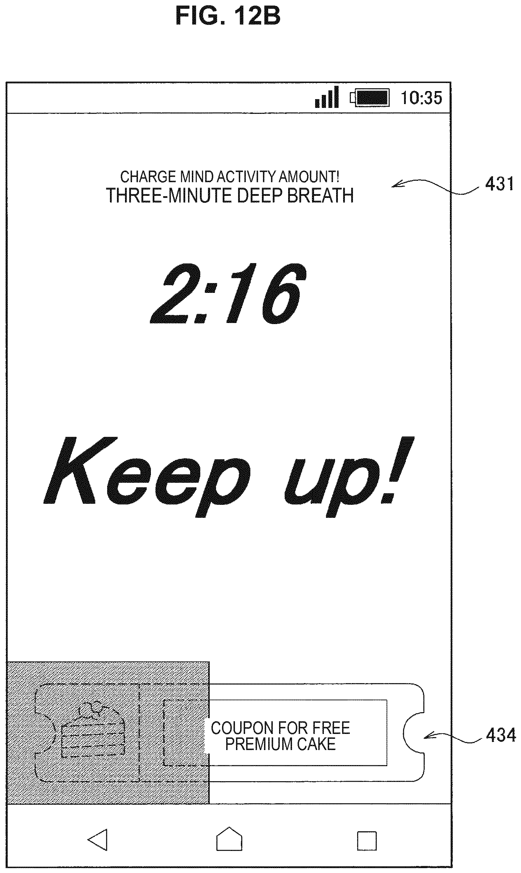

FIGS. 12A to 12D are explanatory diagrams each illustrating an example of the user interface displayed on the portable terminal 200. In a case where a three-minute deep breath is proposed to the user, upon detecting that the user is performing the deep breath to comply the proposal from the information transmitted from the collection device 100, the portable terminal 200 gradually clearly displays the coupon image 434 in accordance with an execution time of the deep breath. However, if the user stops the deep breath in the middle, as shown in FIG. 12B, the portable terminal 200 performs a display indicating that the action of the deep breath is suspended.

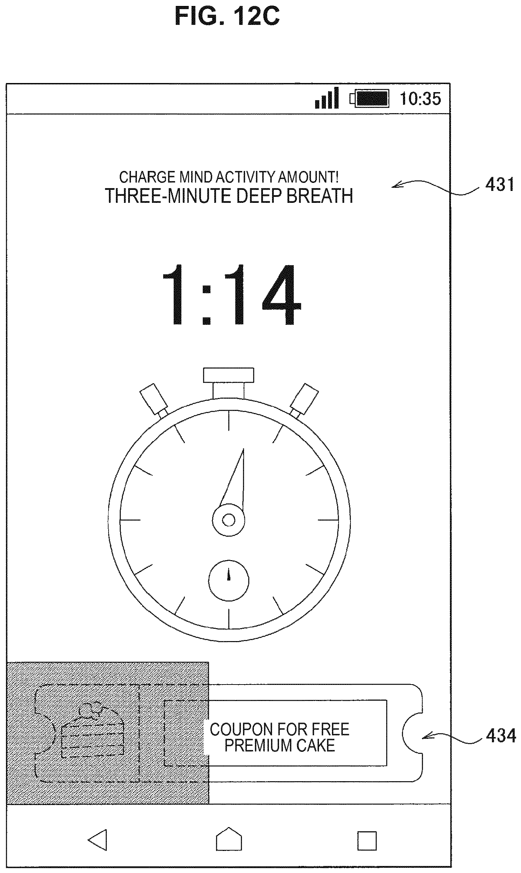

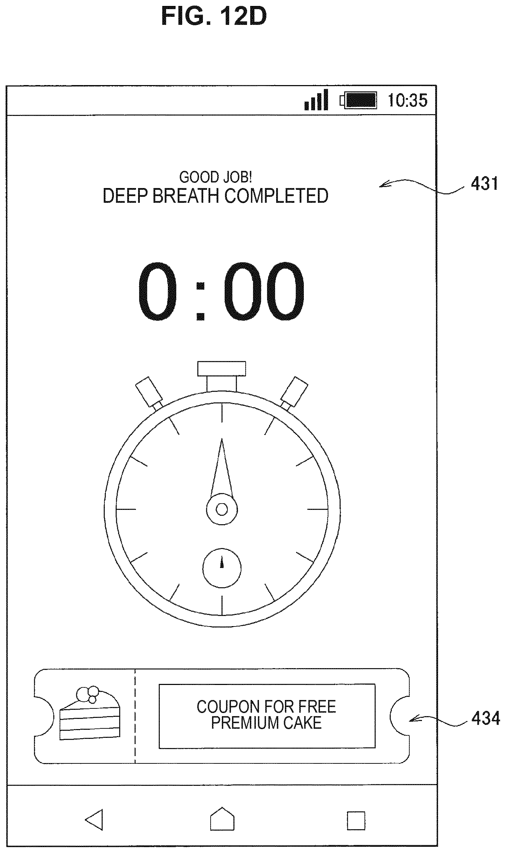

Later, if the user resumes the deep breath, as shown in FIG. 12C, the portable terminal 200 gradually clearly displays the coupon image 434 in accordance with the execution time of the deep breath again. Then, if the user completes the three-minute deep breath, as shown in FIG. 12D, the portable terminal 200 completely clearly displays the coupon image 434.

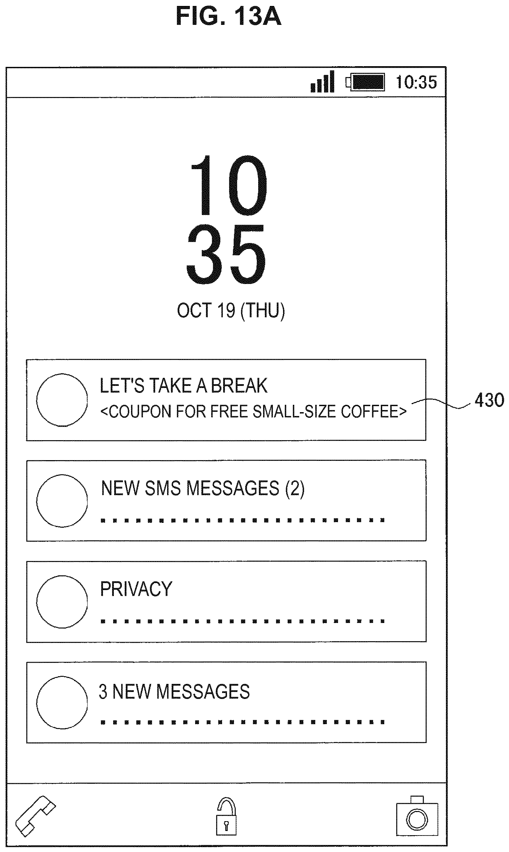

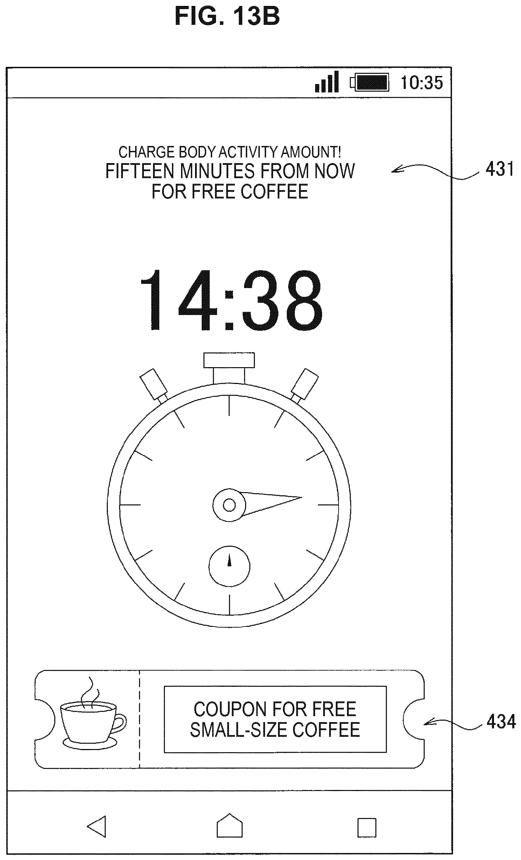

The proposal, which is presented by the portable terminal 200 when the user continues a certain state for a predetermined time, may be a coupon valid only for a limited time. FIGS. 13A and 13B are explanatory diagrams each illustrating an example of the user interface displayed on the portable terminal 200. For example, upon detecting that the tensed state of the user is continued for a predetermined time, the portable terminal 200 performs the push notification as shown in FIG. 13A. If the user selects this push notification, as shown in in FIG. 13B, the portable terminal 200 displays a screen presenting a coupon that offers free coffee valid only for a predetermined time. The portable terminal 200 can cause the user to perform the action for a refreshing feeling by presenting the coupon that offers free coffee valid only for a predetermined time in this manner.

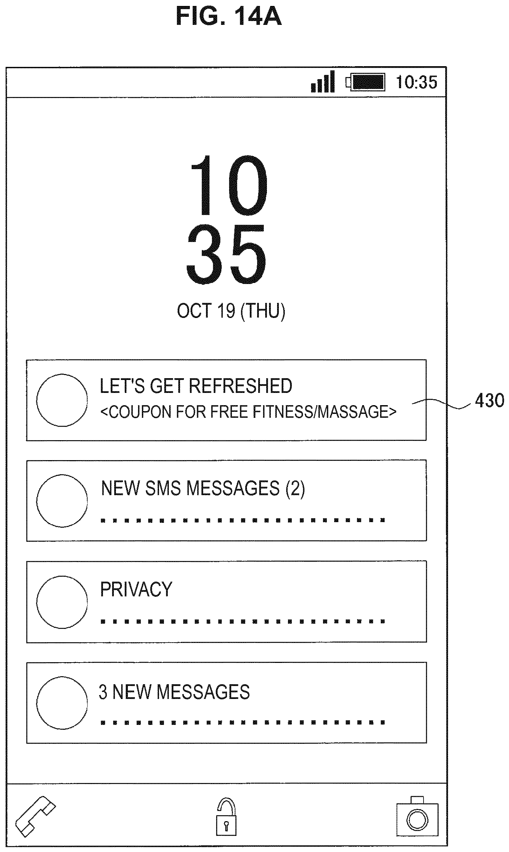

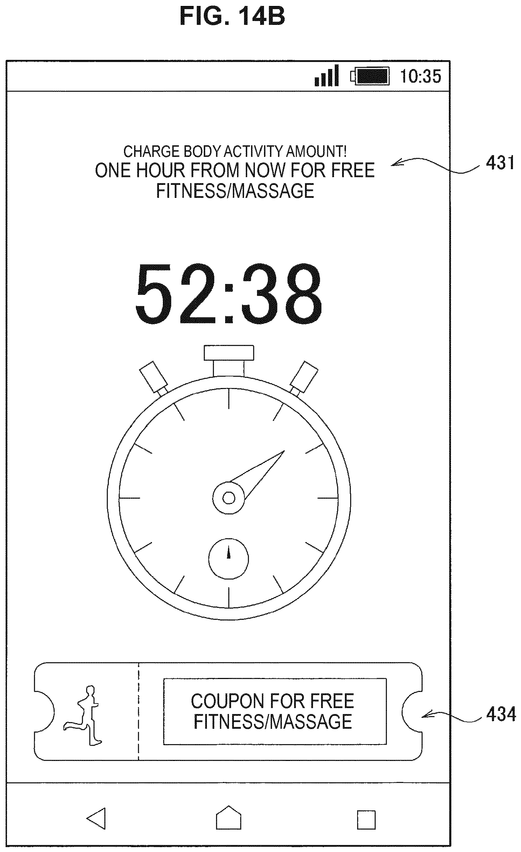

FIGS. 14A and 14B are explanatory diagrams each illustrating an example of the user interface displayed on the portable terminal 200. For example, upon detecting that the tensed state of the user is continued for a predetermined time, the portable terminal 200 performs the push notification as shown in FIG. 14A. If the user selects this push notification, as shown in in FIG. 14B, the portable terminal 200 displays a screen presenting a coupon that offers free massage valid only for a predetermined time. The portable terminal 200 can cause the user to perform the action for a refreshing feeling by presenting the coupon that offers free massage valid only for a predetermined time in this manner.

The portable terminal 200 may change the presentation for changing the state of the user in accordance with the action of the user in the past. For example, if the action of the user in the past indicates that the user has a low physical activity, the portable terminal 200 may present a coupon that offers a free membership for a fitness club valid only for a predetermined time as the presentation for changing the state of the user. Further, for example, if it is determined, from the action of the user in the past, that the user is in the sitting state for a long time and likely has lower back pain, the portable terminal 200 may present a coupon that offers a free membership for a massage clinic valid only for a predetermined time as the presentation for changing the state of the user. Further, for example, if it is determined, from the action of the user in the past, that the user is likely mentally tired, the portable terminal 200 may present a coupon that offers a free movie ticket valid only for a predetermined time as the presentation for changing the state of the user.

An operation example in which a content of the presentation for changing the state of the user is changed in accordance with the action of the user in the past will be described. The portable terminal 200 represents items for determining the state of the user by a point system in advance. Then, the portable terminal 200 reduces the points of each item in accordance with the action of the user and performs the presentation for improving the item having a large amount of point reduction. Initial values of the points can be changed depending on profiles of the users (gender, age, medical history, type of occupation, meal content and time, etc.).

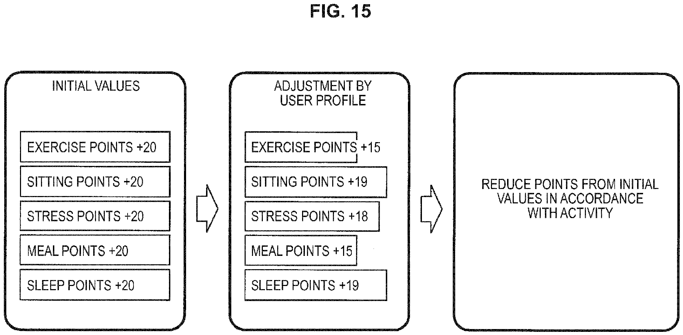

FIG. 15 is an explanatory diagram illustrating an example of how the user items are represented by the point system. In this example, five items, exercise, sitting, stress, meal, and sleep, are represented by the point system. The number and contents of the items are not limited to this example. The initial value of each item has the same value (20 in this example), however, the initial value of each item may have a different value. Further, FIG. 15 shows an example in which each item is changed depending on the user profiles. The points of each user item may be stored in the portable terminal 200 or in the server 300.

Then, the portable terminal 200 reduces the points of each item in accordance with the action performed by the user. For example, if the resting time of the user is continued for a predetermined time, the exercise points and the sitting points decrease by predetermined amounts. Further, for example, if the increased number of steps of the user per predetermined time is a predetermined number or less, the exercise points decrease by the predetermined amount. Further, for example, if the sitting action of the use is continued for a predetermined time, the sitting points decrease by the predetermined amount. Further, for example, if the tensed state of the user is continued for a predetermined time, the stress points decrease by a predetermined amount. Further, for example, if the user skips the breakfast, the meal points decrease by a predetermined amount. Further, for example, if the sleeping time of the user is less than a predetermined time, the sleep points decrease by a predetermined amount. Further, for example, if a predetermined time elapses from the wake-up of the user, the sleep points decrease by the predetermined amount. Further, for example, if the meeting attended by the user continues for a predetermined time or longer, the exercise points, the sitting points, and the stress points decrease by the predetermined amounts.

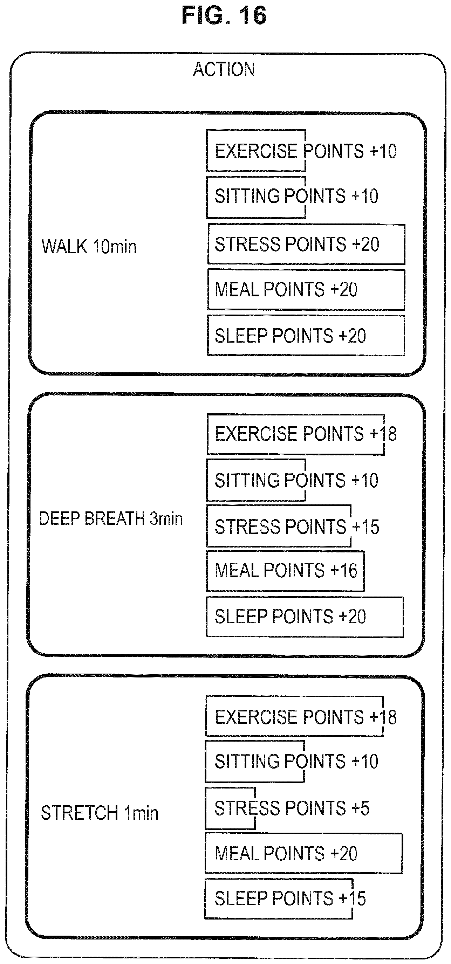

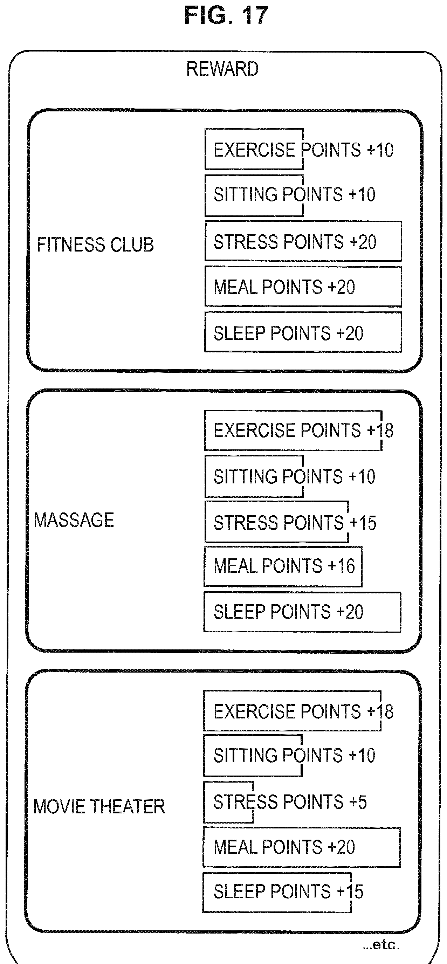

Then, if the points of each user item satisfy a condition, the portable terminal 200 displays a screen presenting the action corresponding to the satisfied condition. FIG. 16 and FIG. 17 are explanatory diagrams each illustrating a correspondence relation between the action to be desirably performed by the user and the condition of the points for presenting such an action. For example, if the exercise points and the sitting points are reduced to 10 points, the portable terminal 200 presents a ten-minute walk to the user. Further, for example, if the exercise points are reduced to 18 points, the sitting points are reduced to 10 points, the stress points are reduced to 15 points, and the meal points are reduced to 16 points, the portable terminal 200 presents a three-minute deep breath to the user. Further, for example, if the exercise points are reduced to 18 points, the sitting points are reduced to 10 points, the stress points are reduced to 5 points, and the sleep points are reduced to 15 points, the portable terminal 200 presents a one-minute stretch to the user.

Further, for example, if the exercise points and the sitting points are reduced to 10 points, the portable terminal 200 presents a coupon for a fitness club to the user. Further, for example, if the exercise points are reduced to 18 points, the sitting points are reduced to 10 points, the stress points are reduced to 15 points, and the meal points are reduced to 16 points, the portable terminal 200 presents a coupon for a massage clinic. Further, for example, if the exercise points are reduced to 18 points, the sitting points are reduced to 10 points, the stress points are reduced to 5 points, and the sleep points are reduced to 15 points, the portable terminal 200 presents a coupon for a movie theater.

The reduced points of each item are reset to the initial value (or the value set in accordance with the user profiles) if the user executes the action presented by the portable terminal 200.

In this manner, the portable terminal 200 can change the content of the presentation for changing the state of the user by representing the state of the user by the point system.

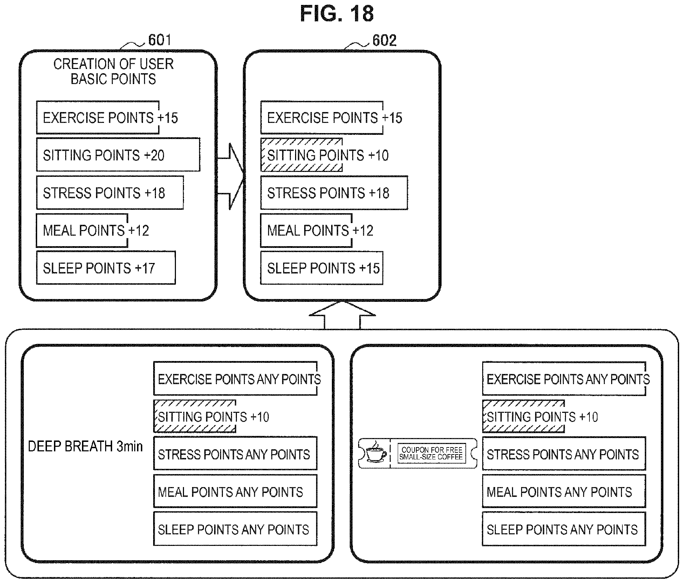

An example of the point change and the proposed action will be described. FIG. 18 is an explanatory diagram illustrating an example of the change in the user's points and the content presented by the portable terminal 200 in accordance with the change in the user's points.

The initial value of the points of each item of one user is hypothetically set as in reference numeral 601 in FIG. 18. That is, hypothetically, the initial value of the exercise points is set to 15 points, the initial value of the sitting points is set to 20 points, the initial value of the stress points is set to 18 points, the initial value of the meal points is set to 12 points, and the initial value of the sleep points is set to 17 points.

If, one day, such a user has a sleeping time of 5 hours, wakes up at six, and stays in the sitting state for 60 minutes after arriving at the workplace, the points of each item of the user change as in reference numeral 602. That is, the sitting points and the seeping points change to 10 points and 15 points, respectively.

When the sitting points change to 10 points, the portable terminal 200 presents a three-minute deep breath as the action to be desirably performed by the user. If it is notified that the user has performed the three-minute deep breath or it is detected that the user has performed the three-minute deep breath by the information from the collection device 100, the portable terminal 200 presents a coupon that offers free coffee to the user and resets the points of each item to the initial value.

In this manner, the portable terminal 200 subtracts the points of each item in accordance with the action performed by the user, and, if the condition is satisfied, the portable terminal 200 can present the action to be desirably performed by the user and also the reward to be provided to the user by performing such an action.

The portable terminal 200 may subtract the points and present the action to be desirably performed by the user using schedule information of the user. The schedule information of the user may be registered in the portable terminal 200 or in the server 300 (or in other servers).

The reward to be provided to the user may vary in accordance with position information. In such a case, the portable terminal 200 may provide a coupon usable in a place slightly away from the user's current location rather than a coupon usable in the closest place for promoting a refreshing feeling of the user. For example, in providing the coupon that offers free coffee, the portable terminal 200 may provide the coupon usable in a place 300 meters away from the user's current location rather than the coupon usable in a place 100 meters away.

### <2. Summary>

As described above, according to the embodiment of the present disclosure, there provided is the portable terminal 200 which can detect the state of the user and, upon determining that a certain state is continued for a predetermined time, present the information for urging the user to change the current state. If the portable terminal 200 determines that the user continues the certain state for a predetermined time, the portable terminal 200 can present the action for changing the ongoing state and the reward, such as the coupon, the earned point, or the like, which is provided to the user once the user performs the presented action.

Steps in the process executed by each apparatus of the present specification need not always be chronologically processed in accordance with the order described as a sequence diagram or a flow chart. For example, steps in the process executed by each apparatus may be processed in an order different from the order described as a flow chart, or may be concurrently processed.

In addition, a computer program for causing hardware such as a CPU, a ROM, and a RAM that is incorporated in each apparatus, to execute a function equivalent to the above-described configuration of each apparatus can also be created. In addition, a storage medium storing the computer program can also be provided. In addition, by forming each functional block illustrated in a functional block diagram, by hardware, a series of processes can also be implemented by hardware.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) A display control device including:
   an acquisition portion that acquires activity information of a user; and
   a display processing portion that performs processing for presenting first information obtained from a state of a user based on the activity information acquired by the acquisition portion and changing a content of the presented first information in accordance with an action of the user.
(2) The display control device according to (1), in which the display processing portion presents the first information and also second information that indicates an action for changing the state of the user.
(3) The display control device according to (2), in which the display processing portion performs the processing for changing the content of the presented first information in accordance with an activity content of the user after presenting the first information.
(4) The display control device according to (3), in which the display processing portion performs the processing for changing the content of the first information in accordance with execution, by the user, of the action indicated as the second information.
(5) The display control device according to any one of (2) to (4), further including a determination portion that determines the state of the user based on the activity information.
(6) The display control device according to (5), in which the display processing portion presents information for changing the state of the user determined by the determination portion as the second information.
(7) The display control device according to (5) or (6), in which the determination portion determines the state of the user using sensing data acquired as the activity information by the acquisition portion.
(8) The display control device according to (7), in which the determination portion determines the state of the user using biological information of the user acquired as the activity information by the acquisition portion.
(9) The display control device according to (7), in which the determination portion determines the state of the user using information regarding a movement of the user acquired as the activity information by the acquisition portion.
(10) The display control device according to (5) to (9), in which the determination portion performs an operation using the activity information to the state of the user represented by a point system.
(11) The display control device according to (10), in which the display control portion presents the first information if points of the user satisfy a predetermined condition.
(12) The display control device according to any one of (1) to (12), in which the display processing portion presents a coupon to be provided to the user as the first information.
(13) The display control device according to (12), in which the display processing portion presents, as the coupon, a coupon that is usable only for a predetermined time.
(14) The display control device according to (13), in which the display processing portion presents, as the coupon, a coupon that is usable only in a predetermined place.
(15) A display control method, including:
   acquiring activity information of a user; and
   performing, by a processor, processing for presenting first information obtained from a state of a user based on the acquired activity information and changing a content of the presented first information in accordance with an action of the user.
(16) A computer program for causing a computer to execute:
   acquiring activity information of a user; and
   performing processing for presenting first information obtained from a state of a user based on the acquired activity information and changing a content of the presented first information in accordance with an action of the user.

### Reference Signs List

- 100: collection device
- 200: portable terminal
- 300: server

## Claims

1. A display control device comprising:
an acquisition portion that acquires activity information of a user; and
a display processing portion that performs processing for presenting first information obtained from a state of a user based on the activity information acquired by the acquisition portion and changing a content of the presented first information in accordance with an action of the user.

2. The display control device according to claim 1, wherein the display processing portion presents the first information and also second information that indicates an action for changing the state of the user.

3. The display control device according to claim 2, wherein the display processing portion performs the processing for changing the content of the presented first information in accordance with an activity content of the user after presenting the first information.

4. The display control device according to claim 3, wherein the display processing portion performs the processing for changing the content of the first information in accordance with execution, by the user, of the action indicated as the second information.

5. The display control device according to claim 2, further comprising a determination portion that determines the state of the user based on the activity information.

6. The display control device according to claim 5, wherein the display processing portion presents information for changing the state of the user determined by the determination portion as the second information.

7. The display control device according to claim 5, wherein the determination portion determines the state of the user using sensing data acquired as the activity information by the acquisition portion.

8. The display control device according to claim 7, wherein the determination portion determines the state of the user using biological information of the user acquired as the activity information by the acquisition portion.

9. The display control device according to claim 7, wherein the determination portion determines the state of the user using information regarding a movement of the user acquired as the activity information by the acquisition portion.

10. The display control device according to claim 5, wherein the determination portion performs an operation using the activity information to the state of the user represented by a point system.

11. The display control device according to claim 10, wherein the display control portion presents the first information if points of the user satisfy a predetermined condition.

12. The display control device according to claim 1, wherein the display processing portion presents a coupon to be provided to the user as the first information.

13. The display control device according to claim 12, wherein the display processing portion presents, as the coupon, a coupon that is usable only for a predetermined time.

14. The display control device according to claim 12, wherein the display processing portion presents, as the coupon, a coupon that is usable only in a predetermined place.

15. A display control method, comprising:
acquiring activity information of a user; and
performing, by a processor, processing for presenting first information obtained from a state of a user based on the acquired activity information and changing a content of the presented first information in accordance with an action of the user.

16. A computer program for causing a computer to execute:
acquiring activity information of a user; and
performing processing for presenting first information obtained from a state of a user based on the acquired activity information and changing a content of the presented first information in accordance with an action of the user.
